(19) **Europäisches Patentamt / European Patent Office / Office européen des brevets**

(11) **EP 3 875 019 A1**

(12) **EUROPEAN PATENT APPLICATION**
published in accordance with Art. 153(4) EPC

(43) Date of publication:
08.09.2021 Bulletin 2021/36

(21) Application number: 19880663.0

(22) Date of filing: 01.11.2019

(51) Int Cl.:
*A61B 1/00* (2006.01)     *A61B 5/06* (2006.01)

(86) International application number:
PCT/JP2019/043042

(87) International publication number:
WO 2020/091045 (07.05.2020 Gazette 2020/19)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 02.11.2018 JP 2018207252

(71) Applicants:
• National Institute of Information and Communications Technology
Koganei-shi,
Tokyo 184-8795 (JP)
• EA Pharma Co., Ltd.
Tokyo 104-0042 (JP)

(72) Inventors:
• TAKIZAWA Kenichi
Koganei-shi, Tokyo 184-8795 (JP)
• KOJIMA Fumihide
Koganei-shi, Tokyo 184-8795 (JP)
• ISHIDA Hirotoshi
Tokyo 104-0042 (JP)
• UMEZAWA Tsutomu
Tokyo 104-0042 (JP)
• TAKEHANA Kenji
Tokyo 104-0042 (JP)

(74) Representative: Rings, Rolf
Klingseisen, Rings & Partner
Patentanwälte
Postfach 10 15 61
80089 München (DE)

(54) **MEDICAL ASSISTANCE SYSTEM**

(57)     PROBLEM TO BE SOLVED: To provide a medical support system that makes an internal terminal smaller and lighter, and that makes it possible to easily estimate the passage, or the range of movement and direction of movement, of an internal terminal in the body, even in an environment with, for example, general medical facilities. MEANS TO SOLVE THE PROBLEM: A first transmitting antenna 40a attached to the body surface transmits the first transmitting wave, and a reflector 5 that is present in the body reflects the first transmitting wave transmitted from the first transmitting antenna 40a. A first receiving antenna 41a attached to the body surface receives the first transmitting wave transmitted from the first transmitting antenna 40a and a reflected wave reflected by the reflector 5, and a location estimation device 2 estimates the passage of the reflector 5 in the body based on changes in the respective phases of the first transmitting wave received by the first receiving antenna 41a and the reflected wave, at time t and time t+$\Delta$t.

FIG. 1

**Description**

**TECHNICAL FIELD**

The present invention relates to a medical support system, which, by means of a transmitting antenna and a receiving antenna attached to the surface of the body, and a reflector that can be placed inside the body, makes it possible to estimate the passage, range of movement and direction of movement of the reflector in the body.

**BACKGROUND ART**

In recent years, when diagnosing digestive diseases or the like, visualizing the activity of the digestive organs provides important materials for diagnosis. As for the method of visualization, a method of swallowing a plurality of radiopaque rings, photographing their locations a plurality of times, with X-rays, at regular intervals, and making a diagnosis based on the distribution of the rings' locations is employed.

In addition, since exposure to X-rays raises a problem when making a diagnosis based on X-rays, a visualization technique to put little burden on the subject is in need. For example, as a method of estimating the location of a capsule endoscope using radio waves or magnetic field has been proposed as a method of estimating the location of an internal terminal. By employing this method, it becomes possible to solve the problem of exposure to X-rays, and make the activity of the digestive organs visible.

Heretofore, as a method of estimating the location in the body using a capsule endoscope, for example, a method of receiving a radio wave transmitted from the capsule endoscope by a plurality of antennas attached to the body surface, and estimating the location in the body from that signal, is disclosed (see, for example, patent literature 1).

In addition, as a method of estimating the location in the body, a method of placing the subject, who has swallowed a capsule endoscope, in a magnetic field generated outside the body, and measuring the amount of magnetic field fluctuations caused by the coil of the capsule endoscope (see, for example, patent literature 2), is known.

**CITATION LIST**

PATENT LITERATURE

Patent Literature 1: Japanese Patent No. 5351356
Patent Literature 2: Japanese Unexamined Patent Application Publication No. 2008-284303

**SUMMARY OF INVENTION PROBLEM TO BE SOLVED BY THE INVENTION**

With the technique disclosed in patent literature 1, the internal terminal has to keep emitting radio waves actively, and therefore the internal terminal needs to have a battery, which makes it difficult to make the internal terminal smaller or lighter. Furthermore, with the technique disclosed in patent literature 2, electric power needs to be supplied from outside, and the subject needs to be placed under a controlled magnetic field, and therefore it is difficult to estimate the location in the body in an environment for medical examination with, for example, general medical facilities.

The present invention has been made in view of the above-described problems, and it is therefore an object of the present invention to provide a medical support system to make it possible to make an internal terminal smaller and lighter, estimate its passage, range of movement and direction of movement in the body easily, even in an environment with, for example, general medical facilities.

**MEANS FOR SOLVING THE PROBLEMS**

The medical support system according to the first invention is a medical support system to estimate the passage of a reflector passing inside the body, and includes a first transmitting antenna that can be attached at least to the body surface, and that transmits a transmitting wave at least at time t and time t+Δt, a first receiving antenna that can be attached at least to the body surface, a reflector that can be placed inside the body, and that reflects the transmitting wave transmitted from the first transmitting antenna, and a location estimation unit that estimates the passage of the reflector in the body, in which the first receiving antenna receives the transmitting wave transmitted from the first transmitting antenna, and the reflected wave of the transmitting wave reflected by the reflector, and in which the location estimation unit estimates the passage of the reflector in the body, based on changes in the respective phases of the transmitting wave received by the first receiving antenna and the reflected wave received by the first receiving antenna, at time t and time t+$\Delta$t.

The medical support system according to a second invention is a medical support system to estimate the movement of a reflector moving inside a body, and includes a first transmitting antenna that can be attached at least to the body surface, and that transmits a transmitting wave at least at time t and time t+$\Delta$t, a first receiving antenna and a second receiving antenna that can be attached at least to the body surface, a reflector that can be placed inside the body, and that reflects the transmitting wave transmitted from the first transmitting antenna, and a location estimation unit that estimates the range of movement and direction of movement of the reflector in the body, in which the first receiving antenna receives the transmitting wave transmitted from the first transmitting antenna, and a reflected wave of the transmitting wave reflected by the reflector, in which the second receiving antenna receives the transmitting wave transmitted from the first transmitting antenna, and the reflected wave of the transmitting wave reflected by the reflector, and in which the location estimation unit estimates the range of movement and direction of movement of the reflector in the body, based on changes in the respective phases of the transmitting wave received by the first receiving antenna and the reflected wave received by the first receiving antenna, at time t and time t+$\Delta$t, and changes in the respective phases of the transmitting wave received by the second receiving antenna and the reflected wave received by the second receiving antenna, at time t and time t+$\Delta$t.

The medical support system according to a third invention is a medical support system to estimate movement of a reflector moving inside a body, and includes a first transmitting antenna and a second transmitting antenna that can be attached at least to the body surface, and that transmit a transmitting wave at least at time t and time t+$\Delta$t, a first receiving antenna that can be attached at least to the body surface, a reflector that can be placed inside the body, and that reflects a first transmitting wave transmitted from the first transmitting antenna, and a second transmitting wave transmitted from the second transmitting antenna, and a location estimation unit that estimates the range of movement and direction of movement of the reflector in the body, in which the first receiving antenna receives each of the first transmitting wave transmitted from the first transmitting antenna, the second transmitting wave transmitted from the second transmitting antenna, a first reflected wave of the first transmitting wave reflected by the reflector, and a second reflected wave of the second transmitting wave, and in which the location estimation unit estimates the range of movement and direction of movement of the reflector in the body, based on changes in the respective phases of the first transmitting wave, the first reflected wave, the second transmitting wave and the second reflected wave, received by the first receiving antenna, at time t and time t+$\Delta$t.

The medical support system according to the fourth invention is a medical support system to estimate movement of a reflector moving inside a body, and includes a first transmitting antenna and a second transmitting antenna that can be attached at least to the body surface, and that transmit a transmitting wave at least at time t and time t+$\Delta$t, a first receiving antenna and a second receiving antenna that can be attached at least to the body surface, a reflector that can be placed inside the body, and that reflects a first transmitting wave transmitted from the first transmitting antenna, and a second transmitting wave transmitted from the second transmitting antenna, and a location estimation unit that estimates the range of movement and direction of movement of the reflector in the body, in which the first receiving antenna receives each of the first transmitting wave transmitted from the first transmitting antenna, the second transmitting wave transmitted from the second transmitting antenna, a first reflected wave of the first transmitting wave reflected by the reflector, and a second reflected wave of the second transmitting wave, in which the second receiving antenna receives each of the first transmitting wave transmitted from the first transmitting antenna, the second transmitting wave transmitted from the second transmitting antenna, the first reflected wave of the first transmitting wave reflected by the reflector, and the second reflected wave of the second transmitting wave, and in which the location estimation unit estimates the range of movement and direction of movement of the reflector in the body, based on changes in the respective phases of the first transmitting wave, the first reflected wave, the second transmitting wave and the second reflected wave, received by the first receiving antenna, at time t and time t+$\Delta$t, and changes in the respective phases of the first transmitting wave, the first reflected wave, the second transmitting wave and the second reflected wave, received by the first receiving antenna, at time t and time t+$\Delta$t.

## ADVANTAGEOUS EFFECTS OF INVENTION

The first invention includes the first transmitting antenna, the first receiving antenna, and the reflector. Consequently, the first receiving antenna can receive the transmitting wave transmitted from the first transmitting antenna at time t and time t+$\Delta$t, and the reflected wave reflected by the reflector. This makes it possible to easily estimate the passage of the reflector in the body even in an environment with, for example, general medical facilities.

In particular, according to the first invention, the reflector reflects the first transmitting wave transmitted from the first transmitting antenna. Consequently, the reflector can reflect the transmitting wave transmitted from the first transmitting antenna through the body. By this means, the reflector does not need to have a battery, and can be made smaller and lighter.

In particular, according to the first invention, the location estimation unit estimates the passage of the reflector.

Consequently, the location estimation unit can identify the changes in the respective phases of the transmitting wave received by the first receiving antenna and the reflected wave received by the first receiving antenna, at time t and time t+Δt. This makes it possible to easily estimate the passage of the reflector in the body even in an environment with, for example, general medical facilities.

The second invention includes a first transmitting antenna, a first receiving antenna, a second receiving antenna, and a reflector. Consequently, the first receiving antenna and the second receiving antenna can receive each of the transmitting wave transmitted from the first transmitting antenna and the reflected wave reflected by the reflector. This makes it possible to accurately and easily estimate the range of movement and direction of movement of the reflector in the body, even in an environment with, for example, general medical facilities.

In particular, according to the second invention, the reflector reflects the first transmitting wave transmitted from the first transmitting antenna. Consequently, each of the first receiving antenna and the second receiving antenna can receive the first transmitting wave transmitted from the first transmitting antenna and the reflected wave reflected by the reflector. This allows the reflector to be smaller and lighter without the need for having a battery.

In particular, according to the second invention, the location measurement unit estimates the range of movement and direction of movement of the reflector. Consequently, based on changes in the respective phases of the first transmitting waves and the reflected waves received respectively by the first receiving antenna and the second receiving antenna, at time t and time t+Δt, the range of movement and direction of movement of the reflector in the body can be estimated. By this means, the range of movement and direction of movement of the reflector in the body can be easily estimated even in an environment with, for example, general medical facilities, and the movement inside the body can be learned.

The third invention includes a first transmitting antenna, a second transmitting antenna, a first receiving antenna, and a reflector. Consequently, the first receiving antenna can receive both transmitting waves transmitted from the first transmitting antenna and the second transmitting antenna, and the reflected waves reflected by the reflector. This makes it possible to accurately and easily estimate the range of movement and direction of movement of the reflector in the body even in an environment with, for example, general medical facilities.

In particular, according to the third invention, the reflector reflects the transmitting waves transmitted from the first transmitting antenna and the second transmitting antenna, respectively. Consequently, the reflector can reflect the first transmitting wave transmitted from the first transmitting antenna and the second transmitting wave transmitted from the second transmitting antenna, while moving in the body. This allows the reflector to be smaller and lighter, without the need for having a battery.

In particular, according to the third invention, the location measurement unit estimates the range of movement and direction of movement of the reflector. Consequently, the location estimation unit can identify the changes in the respective phases of the first transmitting wave, the first reflected wave, the second transmitting wave and the second reflected wave, received by the first receiving antenna, at time t and time t+Δt. As a result, the range of movement and direction of movement of the reflector in the body can be easily estimated even in an environment with, for example, general medical facilities, and the movement inside the body can be learned.

The fourth invention includes a first transmitting antenna, a second transmitting antenna, a first receiving antenna, a second receiving antenna, and a reflector. Consequently, the first receiving antenna and the second receiving antenna can receive the transmitting wave transmitted from the first transmitting antenna and the reflected wave reflected by the reflector, respectively. This makes it possible to more accurately and easily estimate the range of movement and direction of movement of the reflector in the body, even in an environment with, for example, general medical facilities.

In particular, according to the fourth invention, the reflector reflects the transmitting waves transmitted from the first transmitting antenna and the second transmitting antenna, respectively. Therefore, the reflector can reflect the transmitting waves transmitted from each of the first transmitting antenna and the second transmitting antenna, while moving in the body. This allows the reflector to be smaller and lighter without the need for having a battery.

In particular, according to the fourth invention, the location measurement unit estimates the range of movement and direction of movement of the reflector. Consequently, the location estimation unit can identify the changes in the respective phases of the first transmitting wave, the first reflected wave, the second transmitting wave and the second transmitting wave received by the first receiving antenna and the second receiving antenna, respectively, at time t and time t+Δt. By this means, even in an environment with, for example, general medical facilities, the range of movement and direction of movement of the reflector in the body can be estimated more accurately and easily, and the movement inside the body can be learned.

**BRIEF DESCRIPTION OF DRAWINGS**

FIG. 1 is a schematic diagram to show an example of a medical support system according to the present invention;

FIG. 2 is a block diagram to show an example of the configuration of a medical support system according to the present invention;

FIG. 3A is a schematic diagram to show an example of measurement of a reflector at time t in the medical support system according to the first embodiment;

FIG. 3B is a schematic diagram to show an example of measurement of a reflector at time t+Δt in the medical support system according to the first embodiment;

FIG. 4 is a schematic diagram to show an example of measurement of the first antenna pair according to the first embodiment;

FIG. 5 is a schematic diagram to show an example of a measurement result of the first antenna pair according to the first embodiment;

FIG. 6 is a schematic diagram to show an example of measurement of the first to fourth antenna pairs according to another embodiment;

FIG. 7A is a schematic diagram to show an example of a measurement result of the first antenna pair according to another embodiment;

FIG. 7B is a schematic diagram to show an example of a measurement result of the second antenna pair according to another embodiment;

FIG. 7C is a schematic diagram to show an example of a measurement result of the third antenna pair according to another embodiment;

FIG. 7D is a schematic diagram to show an example of a measurement result of the fourth antenna pair according to another embodiment;

FIG. 8 is a schematic diagram to show an example of synthesizing measurement results of the first to fourth antenna pairs;

FIG. 9 is a flowchart to show an example of the operation of reflector measurement according to an embodiment;

FIG. 10A is a schematic diagram to show estimation of the movement of the reflector using the first antenna pair according to the first embodiment;

FIG. 10B is a schematic diagram to show the estimation of the passage of the reflector using the first antenna pair according to the first embodiment; and

FIG. 11 is a schematic diagram to show the estimation of the passage of the reflector according to the fourth embodiment.

## DESCRIPTION OF EMBODIMENTS

Hereinafter, examples of medical support systems and medical support methods according to embodiments of the present invention will be described below with reference to the accompanying drawings.

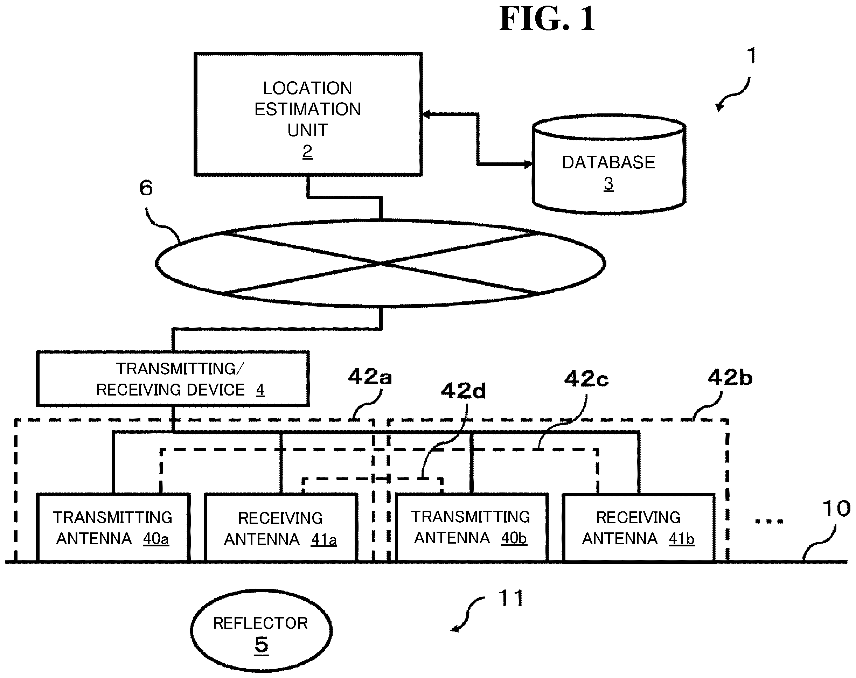

FIG. 1 is a schematic diagram to show an example of a medical support system according to an embodiment of the present invention.

In the medical support system of this embodiment, as shown in FIG. 1, a medical support system 1 is connected to a database 3 and a public communication network 6 (network) by, for example, a location estimation device 2. The location estimation device 2 is connected to a transmitting/receiving device 4 via the public communication network 6. The transmitting/receiving device 4 is connected to a plurality of transmitting antennas 40 and receiving antennas 41. A pair of a transmitting antenna 40 and a receiving antenna 41 constitute an antenna pair 42. The transmitting antennas 40 and the receiving antennas 41 are attached to the body surface 10 of the subject (not shown). A reflector 5 moves inside the body 11 of the subject. The reflector 5 has only to be configured to reflect the radio waves from the transmitting antennas. The reflector 5 may have, for example, an antenna having a predetermined load, or an RFID (Radio Frequency Identifier) function.

< Location estimation device 2 >

The location estimation device 2 is, for example, connected to the transmitting/receiving device 4 via the public communication network 6, and estimates the passage, or the range of movement and direction of movement, of the reflector 5 that is present in the body 11 of the subject (not shown). Furthermore, the location estimation device 2 includes a database 3.

< Database 3 >

The database 3 stores, for example, the baseband waveforms of received waves or reflected waves, algorithms for estimating the location of the reflector 5, past measurement data for location estimation, the subject's MRI images, CT images and so forth, which may provide useful data in location estimation, various logs and so forth, in addition to information about the subject, information about the measurement results, and so forth.

< Transmitting/receiving device 4 >

The transmitting/receiving device 4 controls transmission/receipt by selecting among a plurality of transmitting antennas 40 and receiving antennas 41, attached to the body surface 10 of the subject. The transmitting/receiving device 4 is connected to, for example, each of a first transmitting antenna 40a, a first receiving antenna 41a, a second transmitting antenna 40b, and a second receiving antenna 41b. The transmitting/receiving device 4 may be connected to other transmitting antennas 40 and receiving antennas 41, and may be connected by wire or wireless means. The transmitting/receiving device 4 combines a pair (one set) of a transmitting antenna 40 and a receiving antenna 41 based on, for example, the frequency band set in the transmitting antenna 40 and the receiving antenna 41, their output, the locations they are arranged, and so forth.

For example, if the frequency band is the same, a plurality of transmitting antennas 40 and receiving antennas 41 may be combined in pairs. These transmitting antennas 40 and receiving antennas 41, combined thus, may be stored in the memory of the transmitting/receiving device 4, or in the database 3. The transmitting/receiving device 4 may monitor the conditions of radio waves at the plurality of transmitting antennas 40 and receiving antennas 41 connected, and switch to other transmitting antennas 40 or receiving antennas 41. Furthermore, the transmitting/receiving device 4 converts the received waves, received by the first receiving antenna 41a and the second receiving antenna 41b, into baseband signal waveforms for location estimation in the location estimation device 2.

< Transmitting antenna 40 >

The first transmitting antenna 40a and the second transmitting antenna 40b are controlled by the transmitting/receiving device 4, and, for example, transmits (emits) radio waves of predetermined frequency bands to the first receiving antenna 41a, the second receiving antenna 41b, and the reflector 5. Although the first transmitting antenna 40a and the second transmitting antenna 40b are the first transmitting antenna 40a and the second transmitting antenna 40b in the present embodiment, a plurality of other transmitting antennas may be connected as well. The radio waves to be transmitted from the first transmitting antenna 40a and the second transmitting antenna 40b may be radio waves of the same frequency band, or may be radio waves of different frequency bands.

If, for example, radio waves of the same frequency band are to be transmitted, the first transmitting antenna 40a and the second transmitting antenna 40b may transmit the radio waves at different transmission times. The radio waves, if of different frequency bands, may be transmitted, at the same time, from the transmitting antennas 40 having respective frequency bands. The first transmitting antenna 40a and the second transmitting antenna 40b are attached to the body surface 10 of the subject, and transmit radio waves to the surroundings. The radio waves to be transmitted from the first transmitting antenna 40a and the second transmitting antenna 40b may, for example, have directivity. The directivity of radio waves may be determined by, for example, the antenna angle, the antenna shape and so forth of the first transmitting antenna 40a and the second transmitting antenna 40b.

< Receiving antenna 41 >

The first receiving antenna 41a and the second receiving antenna 41b are controlled by the transmitting/receiving device 4, and receive, for example, radio waves of predetermined frequency bands. Although the first receiving antenna 41a and the second receiving antenna 41b are, for example, the first receiving antenna 41a and the second receiving antenna 41b according to the present embodiment, a plurality of other receiving antennas 41 may be used as well. The first receiving antenna 41a and the second receiving antenna 41b are attached to the body surface 10 of the subject, and receive radio waves of the frequency bands corresponding to the first receiving antenna 41a and the second receiving antenna 41b. In the present embodiment, the first receiving antenna 41a and the second receiving antenna 41b not only receive the radio waves from the first transmitting antenna 40a and the second transmitting antenna 40b, but also receive the reflected waves from the reflector 5 as well.

< Antenna pair 42 >

An antenna pair 42 is set as a pair (set) of a transmitting antenna 40 with a receiving antenna 41 or a reflector 5. According to the present embodiment, the first antenna pair 42a is comprised of, for example, a first transmitting antenna 40a and a first receiving antenna 41a. A second antenna pair 42b is comprised of, for example, a second transmitting antenna 40b and a second receiving antenna 41b. Each antenna pair 42 is attached to the body surface of the subject at a distance. Each antenna pair 42 is attached to the body surface of the subject so as to run parallel to or sandwich the digestive organs in the body, and estimate the movement of the reflector 5, which moves in the digestive organs. When the antenna pairs 42 are attached so as to run parallel to the digestive organs in the body, the movement of the reflector 5 can be identified, whereas, when the antenna pairs 42 are attached so as to sandwich the digestive organs in the body, the reflector 5 passing through the sandwiched portion can be identified.

Note that the antenna pairs 42 may be attached directly to the body surface 10 of the subject. If the antenna pairs 42 can receive the first transmitting wave transmitted by the first transmitting antenna 40a and reflected by the reflector 5, the antenna pairs 42 may be attached, for example, to the surface of clothing or the like. Furthermore, the antenna pairs 42 may be attached integrally with or separately from the transmitting/receiving device 4, and may be connected with the transmitting/receiving device 4 through wired connections or wireless connections. Also, to provide other antenna pairs 42, the first transmitting antenna 40a, the first receiving antenna 41a, the second transmitting antenna 40b, and the second receiving antenna 41b are combined to constitute, for example, a third antenna pair 42c and a fourth antenna pair 42d. The third antenna pair 42c is comprised of, for example, the first transmitting antenna 40a and the second receiving antenna 41b. The fourth antenna pair 42d is comprised of, for example, the second transmitting antenna 40b and the first receiving antenna 41a.

< Reflector 5 >

The reflector 5 is preferably small and lightweight because, for example, the reflector 5 is taken by the subject. Consequently, the reflector 5 may be, for example, about the size of a tablet, and have a smooth surface, so as not to damage the digestive organs. Furthermore, the reflector 5 may be wrapped in a sugar coating or embedded in a tablet, for example. Other shapes are also possible, and the reflector 5 may be enclosed in a capsule, or may be enclosed in a stick package of granules or tablets.

Furthermore, the reflector 5 has a structure capable of having a reflective antenna or an RFID function, for example, and therefore does not have a battery inside. Given a transmitting wave transmitted from a transmitting antenna, the reflector 5 generates a magnetic field using, for example, an induction coil provided inside, generates electric power from the magnetic field generated, and emits a radio wave of a predetermined frequency band as a reflected wave. When a plurality of reflectors 5 are present inside the body of the subject, each reflector 5 may have a different frequency band.

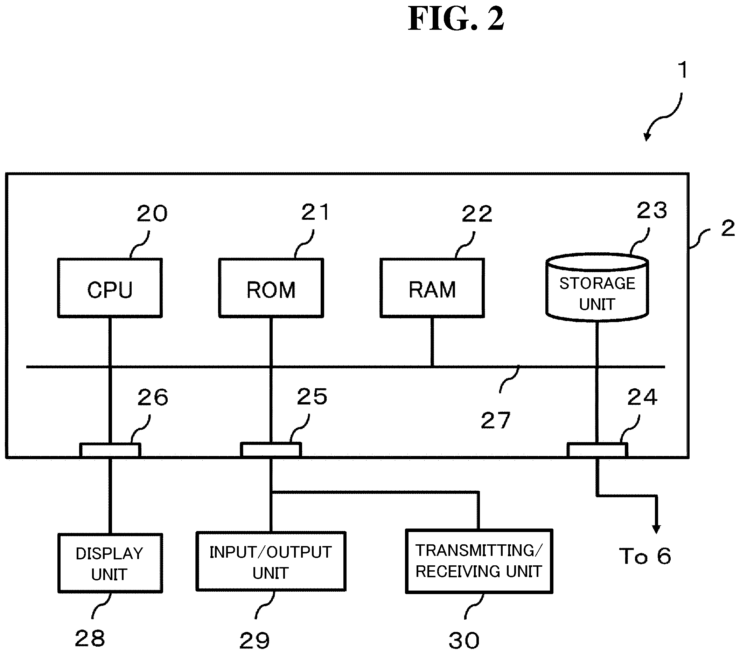

FIG. 2 is a block diagram to show an example of the configuration of a medical support system according to the present invention.

In FIG. 2, a CPU (Central Processing Unit) 20 controls the entire medical support system 1. A ROM (Read Only Memory) 21 stores the operation codes for the CPU 20 in the memory of the ROM 21. A RAM (Random Access Memory) 22 is the work area for use when the CPU 20 runs. For the storage unit 23, for example, apart from an HDD (Hard Disk Drive), a data storage device such as an SSD (solid state drive) is used, and various setting information for running the location estimation device 2, the transmitting/receiving device 4 of the first antenna pair 42a, the first transmitting antenna 40a, the first receiving antenna 41a and the like, programs for processing location estimation, and/or the like are stored. Note that, for example, the location estimation device 2 may have a GPU (Graphics Processing Unit) (not shown). Having a GPU enables arithmetic processing at a higher speed than usual. An I/F 24 is an interface for transmitting and receiving various information to and from the transmitting/receiving device 4 of the first antenna pair 42a, other higher systems, and/or the like, via the public communication network 6.

An I/F 25 is an interface for transmitting/receiving information to and from the input/output unit 29 and the transmitting/receiving unit 30. For the input/output unit 29, for example, a keyboard, another input/output device or the like is used. An I/F 26 is an interface for transmitting and receiving various information to and from a display unit 28, which is, for example, a display.

Note that, as for the functions in each configuration shown in FIG. 1 and FIG. 2, the processes related to the transmission and receipt of radio waves in the transmitting antennas 40 and the receiving antennas 41, the estimation of the passage, or the range of movement and the direction of movement, of the reflector 5 in the location estimation device 2, which will be described later, are implemented as the CPU 20 executes the programs stored in the storage unit 23 or elsewhere by using the RAM 22 as a work area.

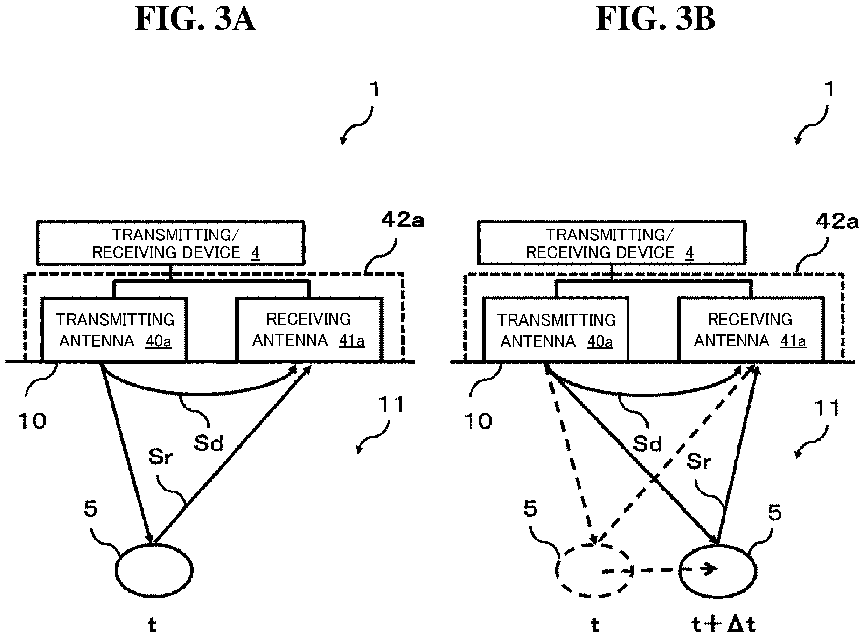

FIG. 3A is a schematic diagram to show an example of measurement of the reflector 5 at time t, in the medical support system 1 according to the first embodiment. Here, assume that the inside of the subject's body is simulated with a cylindrical container filled with a liquid phantom. The electric constants of the liquid phantom are a relative permittivity E = 54.8 and a conductivity σ = 1.05 at 915 MHz.

< Transmission and receipt at time t >

The first transmitting antenna 40a is attached to the body surface 10 of the subject, and transmits the first transmitting wave based on control by the transmitting/receiving device 4. Here, assume that, in the subject's body 11, the reflector 5 taken by the subject in advance is present in the digestive organs in the body.

The first transmitting antenna 40a transmits the first transmitting wave at time t, through a transmission point for transmitting radio waves. Here, the transmitting wave that is transmitted from the transmission point for the first transmitting antenna 40a is a radio wave that constantly has the same frequency band and output. After that, the first transmitting antenna 40a transmits the first transmitting wave, at time t+$\Delta$t, from the transmission point for the first transmitting antenna 40a. The timing for transmitting the first transmitting wave from the first transmitting antenna 40a is controlled by the transmitting/receiving device 4, and assuming, for example, that the moving speed of the reflector 5 in the body is 50 mm or less per second, if estimation is performed 10 times or more per second, estimation error due to phase jump ($\pi$ uncertainty) is not produced. The radio waves to be transmitted from the first transmitting antenna 40a are transmitted, for example, at both time t and time t+$\Delta$t, received as radio waves that arrive directly (direct wave $S_d$) and as reflected waves from the reflector 5 (reflected wave $S_r$).

< Transmission and receipt at time t+$\Delta$t >

FIG. 3B is a schematic diagram to show an example of measurement of the reflector 5 at time t+$\Delta$t in the medical support system 1 according to the first embodiment. Referring to FIG. 3B, too, assume that the first antenna pair 42a is attached to the body surface 10 of the subject in the same manner as in FIG. 3A described above.

The reflector 5 moves, and the first receiving antenna 41a receives both radio waves, namely the direct wave $S_d$ from the first transmitting antenna 40a and the reflected wave $S_r$ from the reflector 5. The transmitting/receiving device 4 receives a composite wave, in which the direct wave Sdt and the reflected wave $S_r$t received by the first receiving antenna 41a are made the direct wave Sdt+the reflected wave $S_r$t. After that, the first receiving antenna 41a receives the radio wave (direct wave $S_d$) that directly arrives from the first receiving antenna 41a and the reflected wave (reflected wave $S_r$) from the reflector 5, at time t+$\Delta$t, as when receiving the first transmitting wave at time t. The first receiving antenna 41a receives the direct wave $S_d$ and the reflected wave $S_r$ at time t+$\Delta$t, following the move of the reflector 5 at time t+$\Delta$t.

< Estimation of movement of reflector 5 >

The first transmitting wave transmitted from the first transmitting antenna 40a is received by the reflector 5, and, from the reflector 5, transmitted into the body as a reflected wave. The reflected wave of the reflector 5 may be transmitted by the RFID function provided in the reflector 5. For example, based on the location in the body at time t, the amount of movement of the reflector 5 in the subject's body up to subsequent time t+$\Delta$t is estimated.

The reflector 5 receives the first transmitting wave transmitted from the first transmitting antenna 40a at time t, followed by the first transmitting wave transmitted by the first transmitting antenna 40a at time t+$\Delta$t. In this case, if the reflector 5 moves between time t and time t+$\Delta$t, let the amount of change of the phase of the reflected wave $S_r$ be $\Delta\theta$, the amount of movement ($\Delta$l) of the reflector 5 can be determined by the following equation:

[Math. 1]

$$\Delta l = \lambda \frac{\Delta\theta}{2\pi}$$

Here, $\lambda$ is the wavelength in the medium of the cylindrical container filled with a liquid phantom. Let the phase constant in the medium be $\beta$, $\lambda$ is given as $\lambda = 2\pi/\beta$. It then follows that the amount of movement ($\Delta$l) of the reflector 5 can be determined from $\Delta\theta/\beta$. The constant $\beta$ is given by the following equation:

[Math. 2]

$$\beta = \omega \sqrt{\frac{\mu \epsilon_r \epsilon_0}{2}} \sqrt{\sqrt{1 + \left(\frac{\sigma_c}{\omega \epsilon_r \epsilon_0}\right)^2} + 1}$$

Here, ω is the angular frequency, μ is the magnetic permeability, $\epsilon_r$ is the relative permittivity, and $\sigma_c$ is the conductivity. Note that, since living tissues are non-magnetic materials, μ is $4\pi \times 10^{-7}$, as in the air. When the transmitting/receiving device 4 uses the 915 MHz band to transmit/receive radio waves, the wavelength (λ) in the liquid phantom that simulates body tissues is 4.4 cm, from the above equation. This principle is applied to the estimation of the location of the reflector 5 in the digestive organs, to estimate the movement of the reflector 5 that is present in the body.

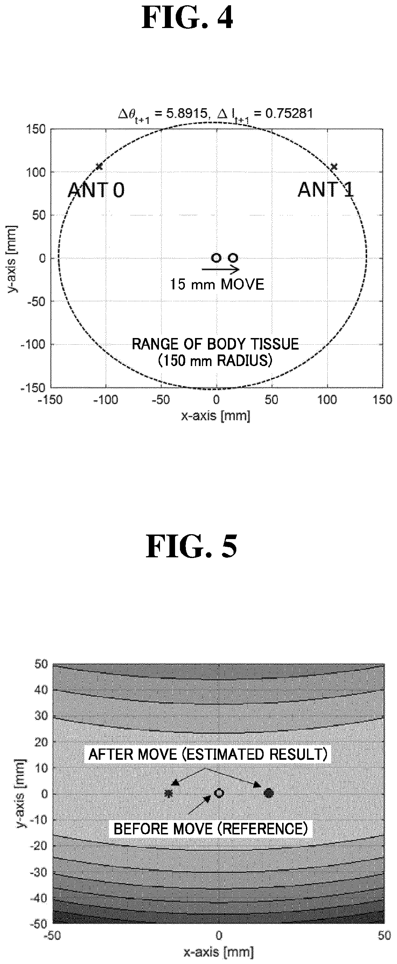

FIG. 4 is a schematic diagram to show an example of measurement of the first antenna pair 42a according to the first embodiment. In FIG. 4, referring back to the description of the configuration shown in FIG. 3, the reflector 5 is the terminal in the digestive organs. The first transmitting antenna 40a transmits the first transmitting wave from a transmission point. When the first transmitting wave that is transmitted is received apart from the first transmitting antenna 40a, the first transmitting antenna 40a is "ANT0", the first receiving antenna 41a is "ANT1", and the range of internal tissues is a 150 mm radius. In this case, given the amount of change in phase $\Delta\theta_{i,j}$ based on observation of the antennas (i, j, i ≠ j) of the first antenna pair 42a, the log-likelihood L (x, y) for a candidate terminal location (x, y) y) is calculated by the following equation:

[Math. 3]

$$L_{i,j}(x, y) = -\frac{\sigma_n^2}{A_{i,j}}(\Delta\theta_{i,j} - \Delta\Theta_{i,j}(x, y))^2$$

Here, $A_{i,j}$ is the amplitude value of the received signal, and $\sigma_n$ is the standard deviation of noise. For the standard deviation σn of noise, the value based on observation of non-signal periods is used. By calculating the likelihood using $A_{i,j}$ and $\sigma_n$ according to the above equation, when there is an amount of change in phase where high received signal power is observed, the likelihood is weighted high. $\Delta\theta_{i,j}$ (x, y) is an expected value of the amount of change in phase with respect to the estimated location (x, y), and can be determined from the following equation, using the above-described amount of movement (Δl).

[Math. 4]

$$\Delta\Theta_{i,j}(x, y) = \beta \cdot \Delta l = \beta \sqrt{(x - x_0)^2 + (y - y_0)^2}$$

Here, (xo, yo) is the coordinates of the reference point for calculating the amount of movement (Δl). This likelihood distribution is shown in FIG. 5.

FIG. 5 is a schematic diagram to show an example of a measurement result of the first antenna pair 42a according to the first embodiment. In the likelihood distribution related to the location of the reflector 5, determined from transmission and receipt between a pair of antennas, locations to show the same likelihood are distributed in an elliptical shape. By this means, it is possible to check on the estimated location after the move at time t+Δt, with reference to the location of the reflector 5 at time t before the move.

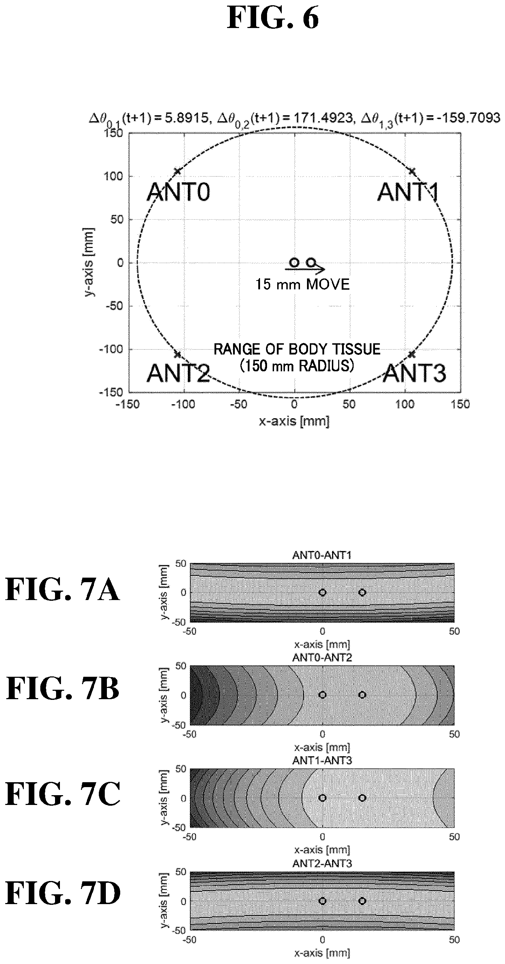

FIG. 6 is a schematic diagram to show an example of measurement of the first to fourth antenna pairs 42 according to another embodiment.

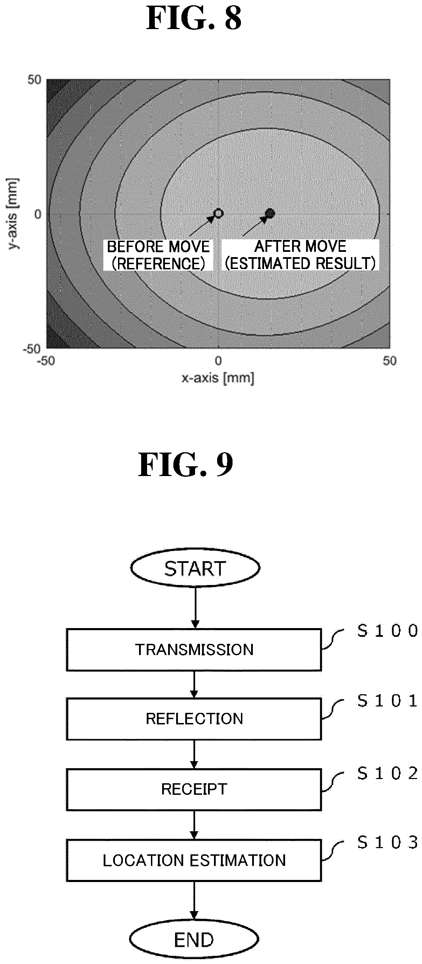

FIG. 6 shows an example in which a plurality of transmitting antennas 40 and receiving antennas 41 are formed into an antenna array and attached to the body surface of the subject. Here, antenna pairs 42 are comprised of transmitting antennas 40 and receiving antennas 41. For example, "ANT0" (first transmitting antenna 40a) and "ANT1" (first receiving antenna 41a) are paired up as a first antenna pair 42a. In this case, to provide another pair of a transmitting antenna 40 and a receiving antenna 41, for example, a second antenna pair 42b is comprised of "ANT0 (first transmitting antenna 40a)" and "ANT2 (second receiving antenna 41b)". Similarly, for example, "ANT3 (second transmitting antenna 40b)" and "ANT1 (first receiving antenna 41a)" may be assigned to a third antenna pair 42c, and, furthermore, "ANT3 (second transmitting antenna 40b)" and "ANT2 (second receiving antenna 41b)" may be assigned to a fourth antenna pair 42d. Given the respective antenna pairs 42 of transmitting antennas 40 and receiving antennas 41, for example, FIGs. 7 and FIG. 8 show the estimation results when, for example, a configuration in which the antenna pairs 42 are formed into an antenna array.

FIGs. 7 show examples of measurement results of the first to fourth antenna pairs 42 according to another embodiment.

FIG. 7A is a schematic diagram to show an example of a measurement result of the first antenna pair 42a ("ANT0" and "ANT1") according to another embodiment. FIG. 7B is a schematic diagram to show an example of a measurement result of the second antenna pair 42b ("ANT0" and "ANT2") according to another embodiment. FIG. 7C is a schematic diagram to show an example of a measurement result of the third antenna pair 42c ("ANT1" and "ANT3") according to another embodiment. FIG. 7D shows an example of a measurement result of the fourth antenna pair 42d ("ANT2" and "ANT3") according to another embodiment.

Next, in FIG. 8, the respective log-likelihoods of the first to fourth antenna pairs 42 measured in FIGs. 7A to 7D are synthesized based on the following equation, to determine the location where the likelihood is the highest.

[Math. 5]

$$L_s(x, y) = \sum_{i,j,i \neq j} L_{i,j}(x, y)$$

$$(\hat{x}, \hat{y}) = \arg \max_{x,y} L_s(x, y)$$

Here, $L_s$ (x, y) is the log-likelihood $L_{i,j}$ (x, y) of a candidate location of the terminal, which is the reflector 5, with respect to the amount of change in phase $\Delta\theta_{i,j}$ based on observation of the antennas (i, j, i ≠ j) of each antenna pair 42 described above. $L_s$ (x, y) calculated here is synthesized to estimate the location where the reflector 5 that is present in the body shows the highest likelihood.

Note that location estimation methods heretofore have conducted measurement in a two-dimensional space using antennas attached to the body surface 10 of the subject, but the same calculation can be used to measure locations in a three-dimensional space.

[Math. 6]

$$L_{i,j}(x, y, z) = -\frac{\sigma_n^2}{A_{i,j}} (\Delta\theta_{i,j} - \Delta\Theta_{i,j}(x, y, z))^2$$

$$L_s(x, y, z) = \sum_{i,j,i \neq j} L_{i,j}(x, y, z)$$

Here, if the reflector 5 is present at the coordinates (x, y, z), the log-likelihood $L_s$ (x, y, x) can be determined.

[Math. 7]

$$(\hat{x}, \hat{y}, \hat{z}) = \arg \max_{x,y,z} L_s(x, y, z)$$

Here, the location where the log-likelihood $L_s$ (x, y, x) is maximized is gained as a measurement result. Note that, when calculating the likelihood, the range (space) of $L_s$ (x, y, z) to be calculated is set so that the amount of change in phase $\Delta\theta$ stays in the range of $[-\pi, +\pi]$. That is, the range of the amount of movement $\Delta l$ handled by the medical support system 1 is $\pi/\beta$ as the upper limit.

FIG. 8 shows an example of synthesizing the likelihood distributions of the first to fourth antenna pairs. The likelihood distributions of the sets of antenna pairs 42 at time t+$\Delta$t after the move are synthesized based on the location of the reflector 5 at time t before the move, so that it becomes possible to accurately estimate the range of movement and direction of movement of the reflector 5 that is present inside the body.

(First embodiment)

The operation of the medical support system 1 according to this embodiment will be described. FIG. 9 is a flowchart to show an example of the operation of the medical support system 1 according to the present embodiment.

The medical support system 1 includes a transmission step 100, a reflection step 101, a receiving step 102, and a location estimation step 103. The medical support system 1 is comprised of a first antenna pair 42a, which is comprised of a first transmitting antenna 40a that can be attached at least to the body surface, and a first receiving antenna 41a that can be attached at least to the body surface, and a reflector 5 that can be placed at least inside the body.

The medical support system 1 is equipped with A, B, and C. a first receiving antenna 41a that can be attached to the body surface 10, a reflector 5 that can be placed inside the body 11 and reflects the first transmitting wave transmitted from the first transmitting antenna 40a, and a location estimation unit that estimates the passage of the reflector 5 in the body 11 by means of the location estimation device 2.

In the first embodiment, one transmitting antenna 40 and one receiving antenna 41 constitute one antenna pair 42. In the first embodiment, a first transmitting antenna 40a and a first receiving antenna 41a constitute a first antenna pair 42a. The first receiving antenna 41a receives the first transmitting wave transmitted from the first transmitting antenna 40a and the reflected wave of the first transmitting wave reflected by the reflector 5. The location estimation unit of the location estimation device 2 estimates the passage of the reflector 5 in the body 11, in the following steps, based on changes in the respective phases of the first transmitting wave and the reflected wave, received by the first receiving antenna 41a, at time t and time t+$\Delta$t.

< Transmission step: S100 >

In the transmission step 100 the first transmitting antenna 40a transmits the first transmitting wave at time t and time t+$\Delta$t. The first transmitting wave is transmitted into the subject's body 11.

< Reflection step: S101 >

In the reflection step 101, the reflector 5 reflects the first transmitting wave transmitted from the first transmitting antenna 40a, at time t and time t+$\Delta$t.

< Receiving step: S102 >

In the receiving step 102, the first receiving antenna 41a receives the first transmitting wave transmitted from the first transmitting antenna 40a and the reflected wave reflected by the reflector 5, at time t and time t+$\Delta$t.

< Location estimation step: S103 >

In the location estimation step 103, given the first transmitting wave received by the first receiving antenna 41a and the reflected wave reflected by the reflector 5, the passage of the reflector 5 in the body is estimated based on changes in their phases at time t and time t+$\Delta$t.

By this means, the operation of the medical support system 1 according to the first embodiment is finished.

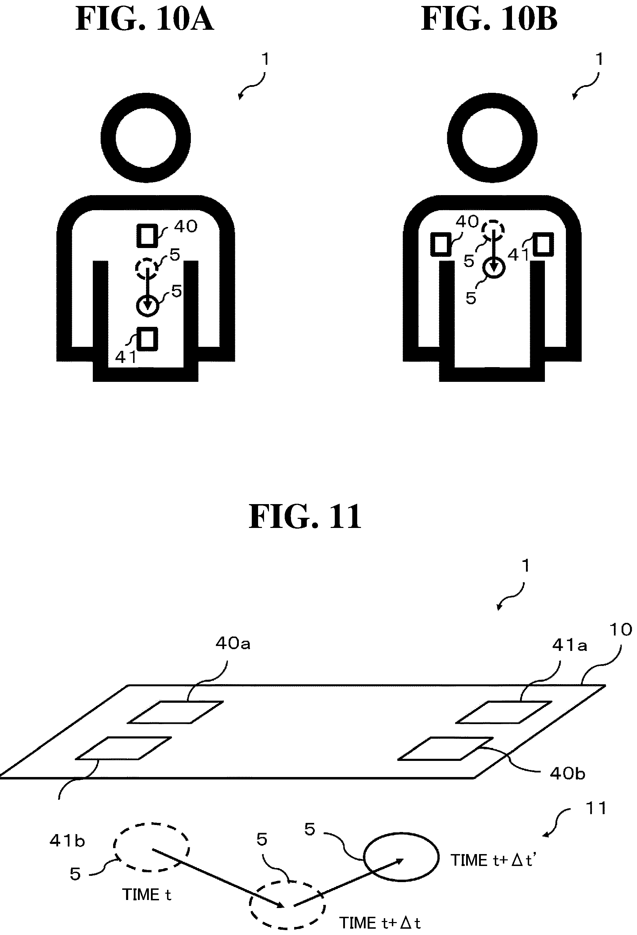

Next, FIG. 10A shows estimation of the movement of the reflector using the first antenna pair 42a according to the first embodiment. Furthermore, FIG. 10B shows estimation of the passage of the reflector using the first antenna pair 42a according to the first embodiment.

FIG. 10A is a schematic view, in which the first antenna pair 42a is attached in a straight line, with respect to the digestive organs, in order to estimate the movement of the reflector 5 in the subject's body 11 (for example, in the digestive organ). For example, at time t, the reflector 5 is in the location in the body indicated by the dotted line, but moves to the location indicated by the solid line at time t+$\Delta$t. By attaching the first antenna pair 42a in the arrangement shown in FIG. 10A, for example, it becomes possible to estimate the movement of the reflector 5 in the body based on the likelihood distribution with respect to the first antenna pair 42a.

FIG. 10B is a schematic view, in which the first antenna pair 42a is attached so as to intersect the digestive organs, in order to estimate the passage of the reflector 5 in the subject's body 11 (for example, in the digestive organs). The reflector 5 is present at the location in the body indicated by the dotted line at time t, but moves to the location indicated by the solid line at time t+$\Delta$t. By attaching the first antenna pair 42a in the arrangement shown in FIG. 10A, it is possible to estimate the passage of the reflector 5 on the line antenna, from the likelihood distribution of change in the phase of the reflected wave at time t and time t+$\Delta$t when the reflector 5 passes through the first antenna pair 42a.

(Second embodiment)

According to the second embodiment, one transmitting antenna 40 and two receiving antennas 41 constitute two sets of antenna pairs 42. With the second embodiment, the operation of the first antenna pair 42a (the first transmitting antenna 40a and the first receiving antenna 41a) and the second antenna pair 42b (the first transmitting antenna 40a and the second receiving antenna 41b) will be described below.

The second embodiment is different from the above-described first embodiment in that the second embodiment has a first transmitting antenna 40a, a first receiving antenna 41a, and a second receiving antenna 41b. The first transmitting antenna 40a and the first receiving antenna 41a form the first antenna pair 42a, and the first transmitting antenna 40a and the second receiving antenna 41b form a second antenna pair 42b. The description of the same configurations as in the above-described embodiment will be omitted here.

Note that, according to the second embodiment, the medical support system 1 includes the first receiving antenna 41a and the first transmitting antenna 40a that can be attached at least to the body surface, and a second antenna pair 42b that is comprised of the first transmitting antenna 40a and the second receiving antenna 41b.

< Transmission step: S100 >

In the transmission step 100, the first transmitting antenna 40a transmits the first transmitting wave, both at time t and time t+$\Delta$t. The first transmitting wave is transmitted into the subject's body 11.

< Reflection step: S101 >

In the reflection step 101, the reflector 5 reflects the first transmitting wave transmitted from the first transmitting antenna 40a at time t and time t+$\Delta$t.

< Receiving step: S102 >

In the receiving step 102, the first receiving antenna 41a in the first antenna pair 42a receives the first transmitting wave transmitted from the first transmitting antenna 40a and the reflected wave reflected by the reflector 5, at time t and time t+$\Delta$t. The second receiving antenna 41b in the second antenna pair 42b receives the first transmitting wave transmitted from the first transmitting antenna 40a and the reflected wave reflected by the reflector 5.

< Location estimation step: S103 >

In the location estimation step 103, the location estimation unit of the location estimation device 2, by synthesizing the likelihood distribution for each set of the antenna pairs 42 based on the first transmitting wave received by the first receiving antenna 41a and the reflected wave reflected by the reflector 5, and the first transmitting wave received by the second receiving antenna 41b and the reflected wave reflected by the reflector 5, estimates the range of movement and direction of movement of the reflector 5 in the body, based on changes in the phases at time t and time t+$\Delta$t.

By this means, the operation of the medical support system 1 according to the second embodiment is finished.

(Third embodiment)

According to a third embodiment, two transmitting antennas 40 and one receiving antenna 41 constitute two antenna pairs 42. With the third embodiment, the operation of the first antenna pair 42a (the first transmitting antenna 40a and the first receiving antenna 41a) and the third antenna pair 42c (the second transmitting antenna 40b and the second receiving antenna 41a) will be described below.

The third embodiment is different from the first and second embodiments described above in that the third embodiment includes a first transmitting antenna 40a, a second transmitting antenna 40b, and a first receiving antenna 41a that can be attached to the body surface. The first transmitting antenna 40a and the first receiving antenna 41a form the first antenna pair 42a. The second transmitting antenna 40b and the first receiving antenna 41a form a second antenna pair 42b. The description of the same configurations as in the above-described embodiment will be omitted here.

The third embodiment includes the first transmitting antenna 40a, the second transmitting antenna 40b, and the first receiving antenna 41a. Consequently, the first receiving antenna 41a can receive transmitting waves transmitted from the first transmitting antenna 40a and the second transmitting antenna 40b, respectively, and their respective reflected waves, reflected by the reflector 5. This makes it possible to accurately and easily estimate the range of movement and direction of movement of the reflector in the body even in an environment with, for example, general medical facilities.

(Fourth embodiment)

According to a fourth embodiment, two transmitting antennas 40 and two receiving antennas 41 constitute four antenna pairs 42. With the fourth embodiment, the operation of each of the first to fourth antenna pairs 42, which are comprised of four pairs of transmitting antennas 40 and receiving antennas 41, will be described below.

The fourth embodiment is different from the first to third embodiments described above in that the fourth embodiment includes two transmitting antennas 40 and two receiving antennas 41. Note that the description of the same configurations as in the above-described embodiment will be omitted here.

The fourth embodiment is comprised of that can be attached at least to the body surface. a first transmitting antenna 40a and a first receiving antenna 41a (first antenna pair 42a), a second transmitting antenna 40b and a second receiving antenna 41b (second antenna pair 42b), the first transmitting antenna 40a and the second receiving antenna 41b (third antenna pair 42c), and the second transmitting antenna 40b and the first receiving antenna 41a (fourth antenna pair 42d).

< Transmission step: S100 >

In the transmission step 100, the first transmitting antenna 40a and the second transmitting antenna 40b transmit the first transmitting wave and the second transmitting wave, at time t and time t+$\Delta$t, respectively. The first transmitting wave and the second transmitting wave are transmitted, for example, into the subject's body 11.

< Reflection step: S101 >

In the reflection step 101, the reflector 5 reflects the first transmitting wave transmitted from the first transmitting antenna 40a and the second transmitting wave transmitted from the second transmitting antenna 40b, at time t and time t+$\Delta$t.

< Receiving step: S102 >

In the receiving step 102, the first receiving antenna 41a receives the first transmitting wave transmitted from the first transmitting antenna 40a and the second transmitting wave transmitted from the second transmitting antenna 40b, at time t and time t+$\Delta$, and their respective reflected waves, reflected by the reflector 5

< Location estimation step: S103 >

In the location estimation step 103, by synthesizing the likelihood distribution for each set of the antenna pairs 42 based on the first transmitting wave received by the first receiving antenna 41a and the reflected wave reflected by the reflector 5, and the second transmitting wave received by the second receiving antenna 41b and the reflected wave reflected by the reflector 5, the range of movement and direction of movement of the reflector 5 in the body is estimated, based on changes in the phases at time t and time t+$\Delta$t.

By this means, the operation of the medical support system 1 according to the fourth embodiment is finished.

Next, FIG. 11 shows estimation of the range of movement range and direction of movement of the reflector according to the fourth embodiment. FIG. 11 is a schematic diagram, in which each antenna pair 42 is attached to the front and rear surfaces of the subject's body, and the range of movement and direction of movement of the reflector 5 that is present in the body 11 are estimated. The reflector 5 moves inside the subject's body 11 at, for example, time t, time t+Δt, and time t+Δt'.

A plurality of antenna pairs 42 may be comprised of, for example, a plurality of transmitting antennas 40 and receiving antennas 41. For example, on the front surface 10 of the subject's body, the first transmitting antenna 40a and the first receiving antenna 41a may constitute the first antenna pair 42a, the first transmitting antenna 40a and the second receiving antenna 41b may constitute a second antenna pair 42b, the second transmitting antenna 40b and the first receiving antenna 41a may constitute a third antenna pair 42c, and the second transmitting antenna 40b and the second receiving antenna 41b may constitute a fourth antenna pair 42d.

Also, the same applies to the rear surface 10 of the subject's body, and, for example, transmitting antennas 40, namely a third transmitting antenna 40c, a third receiving antenna 41c, a fourth transmitting antenna 40d, a fourth receiving antenna 41d, and receiving antennas 41, may be paired and constitute, additionally, the corresponding fifth to eighth antenna pairs 42 (not shown). By constituting these antenna pairs 42, for example, the movement of the reflector 5 at time t and time t+Δt' is measured, and, from the sets of the antenna pairs 42 described above, the likelihood distributions are synthesized based on the transmitting waves and the reflected waves received by the receiving antenna 41 in each of the antenna pairs 42 described above, and the changes in the phases of the reflected waves at each of time t to time t+Δt'. By this means, it becomes possible to estimate the range of movement and direction of movement of the reflector 5 in a three-dimensional space, in the body, at time t to time t+Δt'.

According to the fourth embodiment, it becomes possible to estimate the direction and distance in which the reflector 5 has moved in the body 11, in detail. According to the first modification, for example, even when the subject's body 11 refers to the small intestine to the large intestine, the movement of the reflector 5 is measured in minute detail in chronological order, so that the range of movement and the direction of movement can be estimated in more detail from the path of that move.

Furthermore, according to the present embodiment, the reflector 5 receives radio waves from outside the body, and reflects the received radio waves. Consequently, the reflector 5 does not need to have a built-in battery and responds only when necessary. By this means, for example, the passage, or the range of movement and direction of movement, of the reflector 5 in the body, can be measured only upon medical examination.

Furthermore, according to the present embodiment, the transmitting/receiving device 4 may switch the radio wave output of the transmitting antenna 40 or the type of the antenna to be made to respond. Consequently, the transmitting/receiving device 4 can receive or adjust the radio waves depending on, for example, which part of the digestive organs of the subject is to be diagnosed. By this means, the location estimation device 2 can measure the reflector 5 depending on the diagnosis location of the subject. Furthermore, even when a plurality of reflectors 5 having different frequency bands are present in the body of the subject, the transmitting/receiving device 4 can quickly measure the passage, or the range of movement and direction of movement, of other reflectors 5, by switching the frequency band of the transmitting/receiving device 4. This makes it possible to diagnose different symptoms and digestive organs at the same time.

Furthermore, according to the present embodiment, the transmitting/receiving device 4, the transmitting antennas 40, and the receiving antennas 41 may be, for example, configured wearable. Consequently, the transmitting antennas 40 and the receiving antennas 41 can be kept attached to the body surface as antenna pairs 42. By this means, the subject can, for example, diagnose the digestive organs' capacity for movement in a normal living environment.

Furthermore, according to the present embodiment, for example, the transmitting/receiving device 4 may include a memory. Consequently, the log of the movement of the reflector 5 can be temporarily recorded in the memory inside the transmitting/receiving device 4. By this means, for example, the passage, or the range of movement and direction of movement, of the reflector 5 in the subject's body, can be continuously measured and recorded even while no connection is established with the location estimation device 2.

Although some of the embodiments of the present invention have been described above, these embodiments have been presented simply as examples, and are by no means intended to limit the scope of the present invention. These novel embodiments can be implemented in a variety of other forms, and can be omitted, replaced, or changed in a variety of ways without departing from the gist of the present invention. These embodiments and modifications are included in the scope and gist of the present invention, as well as in the scope of the invention recited in the claims and any equivalent of the invention recited in the claims.

**REFERENCE SIGNS LIST**

| 1: | medical support system |
|---|---|
| 10: | body surface |
| 11: | inner body |
| 2: | location estimation device |
| 20: | CPU |
| 21: | ROM |
| 22: | RAM |
| 23: | storage unit |
| 24: | I/F |
| 25: | I/F |
| 26: | I/F |
| 27: | internal bus |
| 28: | display |
| 29: | input/output unit |
| 3: | database |
| 30: | transmitting/receiving unit |
| 4: | transmitting/receiving device |
| 40: | transmitting antenna |
| 40a: | first transmitting antenna |
| 40b: | second transmitting antenna |
| 40c: | third transmitting antenna |
| 40d: | fourth transmitting antenna |
| 41: | receiving antenna |
| 41a: | first receiving antenna |
| 41b: | second receiving antenna |
| 41c: | third receiving antenna |
| 41d: | fourth receiving antenna |
| 42: | antenna pair |
| 42a: | first antenna pair |
| 42b: | second antenna pair |
| 42c: | third antenna pair |
| 42d: | fourth antenna pair |
| 5 : | reflector |
| 6: | public communication network |
| $S_d$: | direct wave |
| $S_r$: | reflected wave |

**Claims**

1. A medical support system to estimate passage of a reflector passing inside a body, the medical support system comprising:

   a first transmitting antenna that can be attached at least to a body surface, and that transmits a transmitting wave at least at time t and time t+$\Delta$t;
   a first receiving antenna that can be attached at least to the body surface;
   a reflector that can be placed inside the body, and that reflects the transmitting wave transmitted from the first transmitting antenna; and
   a location estimation unit that estimates the passage of the reflector in the body,
   wherein the first receiving antenna receives

      the transmitting wave transmitted from the first transmitting antenna, and
      the reflected wave of the transmitting wave reflected by the reflector, and

   wherein the location estimation unit estimates the passage of the reflector in the body, based on changes in phases of the transmitting wave received by the first receiving antenna and the reflected wave received by the first receiving antenna, at time t and time t+$\Delta$t.

2. A medical support system to estimate movement of a reflector moving inside a body, the medical support system comprising:

a first transmitting antenna that can be attached at least to a body surface, and that transmits a transmitting wave at least at time t and time t+Δt;
a first receiving antenna and a second receiving antenna that can be attached at least to the body surface;
a reflector that can be placed inside the body, and that reflects the transmitting wave transmitted from the first transmitting antenna; and
a location estimation unit that estimates a range of movement and direction of movement of the reflector in the body,
wherein the first receiving antenna receives

the transmitting wave transmitted from the first transmitting antenna, and
a reflected wave of the transmitting wave reflected by the reflector, wherein the second receiving antenna receives
the transmitting wave transmitted from the first transmitting antenna, and
the reflected wave of the transmitting wave reflected by the reflector, and

wherein the location estimation unit estimates the range of movement and direction of movement of the reflector in the body, based on

changes in respective phases of the transmitting wave received by the first receiving antenna and the reflected wave received by the first receiving antenna, at time t and time t+Δt, and
changes in respective phases of the transmitting wave received by the second receiving antenna and the reflected wave received by the second receiving antenna, at time t and time t+Δt.

3. A medical support system to estimate movement of a reflector moving inside a body, the medical support system comprising:

a first transmitting antenna and a second transmitting antenna that can be attached at least to a body surface, and that transmit a transmitting wave at least at time t and time t+Δt;
a first receiving antenna that can be attached at least to the body surface;
a reflector that can be placed inside the body, and that reflects a first transmitting wave transmitted from the first transmitting antenna, and a second transmitting wave transmitted from the second transmitting antenna; and
a location estimation unit that estimates the range of movement and direction of movement of the reflector in the body,
wherein the first receiving antenna receives each of the first transmitting wave transmitted from the first transmitting antenna, the second transmitting wave transmitted from the second transmitting antenna, a first reflected wave of the first transmitting wave reflected by the reflector, and a second reflected wave of the second transmitting wave, and
wherein the location estimation unit estimates the range of movement and direction of movement of the reflector in the body, based on changes in respective phases of the first transmitting wave, the first reflected wave, the second transmitting wave and the second reflected wave, received by the first receiving antenna, at time t and time t+Δt.

4. A medical support system to estimate movement of a reflector moving inside a body, the medical support system comprising:

a first transmitting antenna and a second transmitting antenna that can be attached at least to a body surface, and that transmit a transmitting wave at least at time t and time t+Δt;
a first receiving antenna and a second receiving antenna that can be attached at least to the body surface;
a reflector that can be placed inside the body, and that reflects a first transmitting wave transmitted from the first transmitting antenna, and a second transmitting wave transmitted from the second transmitting antenna; and
a location estimation unit that estimates a range of movement and direction of movement of the reflector in the body,
wherein the first receiving antenna receives each of the first transmitting wave transmitted from the first transmitting antenna, the second transmitting wave transmitted from the second transmitting antenna, a first reflected wave of the first transmitting wave reflected by the reflector, and a second reflected wave of the second transmitting wave,

wherein the second receiving antenna receives each of the first transmitting wave transmitted from the first transmitting antenna, the second transmitting wave transmitted from the second transmitting antenna, the first reflected wave of the first transmitting wave reflected by the reflector, and the second reflected wave of the second transmitting wave, and

wherein the location estimation unit estimates the range of movement and direction of movement of the reflector in the body, based on

changes in respective phases of the first transmitting wave, the first reflected wave, the second transmitting wave and the second reflected wave, received by the first receiving antenna, at time t and time t+Δt, and changes in respective phases of the first transmitting wave, the first reflected wave, the second transmitting wave and the second reflected wave, received by the first receiving antenna, at time t and time t+Δt.

# FIG. 1

EP 3 875 019 A1

# FIG. 2

EP 3 875 019 A1

# FIG. 3A

**42a**

| TRANSMITTING/ RECEIVING DEVICE 4 |
|---|

| TRANSMITTING ANTENNA 40a | RECEIVING ANTENNA 41a |

10

11

Sd

Sr

5

t

# FIG. 3B

**42a**

| TRANSMITTING/ RECEIVING DEVICE 4 |
|---|

| TRANSMITTING ANTENNA 40a | RECEIVING ANTENNA 41a |

10

11

Sd

Sr

5

5

t

t+Δt

EP 3 875 019 A1

# FIG. 4

# FIG. 5

# FIG. 6

$\Delta\theta_{0,1}(t+1) = 5.8915, \Delta\theta_{0,2}(t+1) = 171.4923, \Delta\theta_{1,3}(t+1) = -159.7093$

**FIG. 7A**

ANT0-ANT1

**FIG. 7B**

ANT0-ANT2

**FIG. 7C**

ANT1-ANT3

**FIG. 7D**

ANT2-ANT3

# FIG. 8

# FIG. 9

# FIG. 10A

# FIG. 10B

# FIG. 11

## INTERNATIONAL SEARCH REPORT

| International application No. |
| --- |
| PCT/JP2019/043042 |

### A. CLASSIFICATION OF SUBJECT MATTER

A61B 1/00(2006.01)i; A61B 5/06(2006.01)i
FI: A61B5/06; A61B1/00 552

According to International Patent Classification (IPC) or to both national classification and IPC

### B. FIELDS SEARCHED

Minimum documentation searched (classification system followed by classification symbols)
A61B1/00; A61B5/06-5/07; A61M25/095; G01S5/00-5/14; G01S13/00-13/72

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2016-539676 A (ROCK WEST MEDICAL DEVICES, LLC) 22.12.2016 (2016-12-22) paragraphs [0034], [0038], [0040]-[0041], [0044], [0096]-[0098], fig. 1A, 1B, 2A, 12 | 1-4 |
| A | JP 2004-219329 A (NTT DOCOMO INC.) 05.08.2004 (2004-08-05) paragraphs [0023]-[0075], fig. 1-11 | 1-4 |
| A | JP 2011-509106 A (SIEMENS AG) 24.03.2011 (2011-03-24) paragraphs [0014]-[0040], fig. 1-4 | 1-4 |

☐ Further documents are listed in the continuation of Box C.  ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14 January 2020 (14.01.2020) | 28 January 2020 (28.01.2020) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

Information on patent family members

| International application No. |
| --- |
| PCT/JP2019/043042 |

| Patent Documents referred in the Report | Publication Date | Patent Family | Publication Date |
| --- | --- | --- | --- |
| JP 2016-539676 A | 22 Dec. 2016 | US 2015/0112189 A1 paragraphs [0034], [0038], [0040]-[0041], [0044], [0100]-[0102], fig. 1A, 1B, 2A, 12 WO 2015/061343 A1 CA 2927687 A1 | |
| JP 2004-219329 A | 05 Aug. 2004 | US 2004/0210131 A1 paragraphs [0028]-[0100], fig. 1-11 EP 1440659 A2 EP 1829478 A2 CN 1517717 A | |
| JP 2011-509106 A | 24 Mar. 2011 | US 2010/0274086 A1 paragraphs [0019]-[0045], fig. 1-4 WO 2009/083409 A1 DE 102008003005 A1 CN 101909519 A | |

Form PCT/ISA/210 (patent family annex) (January 2015)

**EP 3 875 019 A1**

**REFERENCES CITED IN THE DESCRIPTION**

**Patent documents cited in the description**

- JP 5351356 B

- JP 2008284303 A